# EUROPEAN PATENT APPLICATION

(11) **EP 0 628 315 A1**
(43) Date of publication of application: **14.12.1994**
(21) Application number: 94202245.0
(22) Date of filing: 18.03.1987
(51) Int. Cl.: A61K 47/48, C07K 15/00

(54) **Lectin complex and probe and method for making same**

(30) Priority: 20.03.1986 US 841551
(62) Divisional of application: 87902922.1
(71) Applicant: DANA-FARBER CANCER INSTITUTE, INC., Boston, MA 02115 (US)
(72) Inventor: Moroney, Simon E, 80805 Munich (DE); Blattler, Walter A, Brookline, MA 02146 (US); Lanbert, John M, Cambridge, Ma 02139 (US); D Alarcao, Linda J, Jamaica Plain, MA 02130 (US)
(74) Representative: Rickard, Timothy Mark Adrian

(57) **Abstract**

A cytotoxic lectin complex having diminished non-specific toxicity comprising a cytotoxic lectin covalently bonded to a specific ligand comprising a disaccharide or higher polysaccharide derivative capable of binding specifically to an oligosaccharide-binding site on the lectin and a monoclonal antibody covalently bonded to the cytotoxic lectin complex.

## Description

This invention relates to a method of making a cytotoxic lectin complex having diminished non-specific toxicity as well as to a novel ligand-support complex or probe useful in making such a cytotoxic lectin complex.

### Background of the Invention

There are several known cytotoxic lectins such as ricin, abrin, modeccin, volkensin and viscumin which kill eucaryotic cells very efficiently. These lectins are heterodimeric proteins containing two subunits linked by a disulfide bond. One subunit, denominated the A-chain, exhibits cytotoxic activity by catalytic inactivation of ribosomes; while the other, the B-chain, contains binding sites specific for galactose-terminated oligosaccharides. Since molecules containing oligosaccharide components are ubiquitous on cell surfaces, the lectins are non-discriminating, hence non-specific in their cytotoxicity. This characteristic greatly limits their utility for killing selected diseased or abnormal cells such as tumor cells.

Previously, the lectin has been cleaved into its two subunits, and the A chain alone linked to a monoclonal antibody to provide a toxin having specific activity, but such conjugated products display significantly reduced toxicity as compared to the whole lectin.

It has also been proposed to block the oligosaccharide binding sites of a lectin, concanavalin A, by treating it with a photoactivatable aryl azido derivative of mannose which binds specifically to the concanavalin A binding sites, then exposing to ultraviolet light to form a covalent bond between the concanavalin A and the polysaccharide derivative, as described by Beppu et. al., J. Biochem. 78, 1013-1019 (1975). The product retained activity at two of its four binding sites and displayed reduced haemagglutinating activity. Similar results were recorded by Fraser et al., Proc. Nat. Acad. Sci. (USA) 73, 790-794 (1976) using succinylated concanavalin A, and by Thomas, Methods Enzymol. 46, 362-414 (1977) in an analogous procedure. Similarly, Baenziger et al., J. Biol. Chem. 257, 4421-4425 (1982) described treating the lectins concanavalin A, ricin, and lectin from liver, with appropriate photoactivatable derivatives of glycopeptides, then exposing to light to form a covalent bond between the lectin and the glycopeptide derivative. However, only 1-2% incorporation of the glycopeptide derivative was achieved in each case. There was, apparently, no attempt to determine the efficacy of the labelling of the lectins through measurements of either cytotoxicity (in the case of ricin) or of haemagglutination (in the case of concanavalin A). An analogous procedure has been employed using ricin and a photoactivatable derivative of galactose as described by Houston, J. Biol. Chem. 258, 7208-7212 (1983). The product was found to be 280 times less toxic toward cells than untreated ricin, although the A chain alone showed full activity in the inhibition of protein synthesis in cell lysates.

Thorpe et. al., Eur. J. Biochem. 140, 63-71 (1984) described conjugating a monoclonal antibody to intact ricin, then fractionating the product by affinity chromatography to isolate in 20% yield a fraction in which the monoclonal antibody by chance blocked the oligosaccharide binding sites of the ricin thereby diminishing the conjugate's capacity to bind non-specifically to cells.

The variability in end results using photoactivatable polysaccharides or monoclonal antibodies as blocking agents for the oligosaccharide binding sites of lectins is eliminated by the present invention which provides effective and reproducible reduction of the non-specific toxicity of lectins without excessive reduction of their cytotoxic properties.

The present invention comprises a method of making a cytotoxic lectin having diminished nonspecific toxicity which comprises providing a specific ligand for the oligosaccharide-binding sites on said lectin, covalently linking said ligand to a solid support to form a probe, bringing said lectin into contact with said probe through a specific interaction between said binding sites and said ligand, and forming a covalent bond between said lectin and said ligand thereby providing a complex of said lectin and said ligand covalently linked to said support. The present invention also comprises the additional step of severing the covalent linkage between the ligand and the support to release a complex comprising the lectin covalently bonded to the ligand. It also comprises the further step of forming a covalent bond between the complex and a monoclonal antibody. Other and further features will be apparent from the description which follows.

The method of the present invention can be used with any of the lectins having cytotoxic activity such as ricin, abrin, modeccin, volkensin, or viscumin.

In the drawings,
Fig. 1 is a schematic representation of the reactions leading to the production of the activated ligand-support probe of Example 1;
Fig. 2 is a schematic representation of the reactions leading to the production of the activated ligand-support probe of Example 2; and
Figs. 3A and 3B are a schematic representation of the reactions leading to the production of the activated ligand-support probe of Example 3.

The solid support employed in this invention may be any of the solid supports commonly used for affinity chromatography, such as cross-linked polyacrylamide and derivatives thereof, for example aminoethyl polyacrylamide, derivatised porous glass-beads, latex beads, polyvinyl alcohol beads, and the like.

The ligand employed in the method may be any compound which binds specifically to the oligosaccharide binding sites of lectins. Preferably the ligand contains a polysaccharide group such as a disaccharide or higher polysaccharide and it preferably contains a galactose moiety for optimum binding capacity.

The covalent linkage between the ligand and the support to provide the probe can be formed in any conventional manner. Any suitable heterobifunctional cross-linking agents, many of which are known and commercially available, can be used. The cross-linking agent must be cleavable when subjected to selected conditions or reagents to allow separation from the support of the ligand-lectin complex after it is formed, although in some cases the complex may remain bonded to the support during subsequent use. Alternatively one or both of the support and the ligand may be modified to include a functional group reactive with the other to form a covalent linkage between the ligand and the support, avoiding the need for using a discrete crosslinking agent. The moiety providing cleavability of the ligand from the support to which it is covalently bonded may take the form of a disulfide group preferentially cleavable by reduction under selected conditions under which the internal disulfide linkage of the lectin itself is not so readily cleaved, a thioester group cleavable by aminolysis, an azo group cleavable by reduction preferentially with dithionite, an ortho-nitrobenzyl ester or ortho-nitrobenzyl carbamate cleavable by light of a wavelength of about 360 nm, a vicinal glycol group cleavable by oxidation with periodate, or the like.

Similarly the covalent bond between the ligand and the lectin can be formed by conventional chemical procedures for bonding proteins or polypeptides to other compounds or groups, for example by using appropriate cross-linking agents.

The covalent bond between the ligand and the support as well as the covalent bond between the ligand and the lectin can each be formed in two or more successive stages, if desired. For example, 2-pyridyldithiopropionic acid can be reacted with a support such as aminoethyl-polyacrylamide to provide a support having 2-pyridyldithio groups bonded to it. A ligand containing a disaccharide moiety can be prepared by modifying lactose to form N-(2'-mercaptoethyl) lactamine, which can then replace the 2-pyridyl groups on the support by disulfide interchange, and the residual amine group of the lactamine can then be employed as a functional group to form a covalent bond with the lectin. This can be accomplished by using a bifunctional cross-linking agent such as 2,4-dichloro-6-methoxy triazine, one chlorine atom or which is very reactive with amino groups at pH 8, the reaction proceeding to completion in minutes; the remaining chlorine atom being reactive with amino groups much more slowly, requiring 24 hours or more at a pH of 8.5 or more. This cross-linking agent can be first reacted with the residual amino group of the lactamine at pH 8, and subsequently reacted at higher pH with an amino group of the lectin after it has been properly positioned by specific binding of the lectin to the galactose moiety of the lactamine.

This second cross-linking agent need not be cleavable since it is desired that the ligand remain permanently bonded to the lectin to block the oligosaccharide binding sites of the latter. In another embodiment the ligand itself can be chemically modified to include a functional group reactive with lectins to form a covalent bond therewith.

In the case where the ligand itself is modified to include two functional groups, one capable of forming the covalent linkage with the support, another capable of forming a covalent linkage to the lectin, the two functional groups are preferably different in reactivity or conditions of reaction, so that the ligand can be linked to the support under one set of conditions, and bonded to the lectin under another set of conditions. In this case, the ligand itself may be regarded as a heterobifunctional cross-linking agent containing a di-or polysaccharide moiety capable of binding specifically to the oligosaccharide binding sites of lectins. The two functional groups incorporated in the ligand may be the same as those present in any conventional hetero-bifunctional cross-linking agent.

After the ligand has been linked to the solid support to form a probe, the lectin has been positioned on it by specific binding and then covalently bonded to it to form a ligand-lectin complex, the complex may if desired be severed from the support by a cleavage of the ligand-support linkage by conventional procedures.

The complex, either free or still linked to the solid support, has its oligosaccharide binding sites blocked by the ligand, consequently it no longer displays non-specific or non-selective binding to cells and has lost much of its non-specific toxicity. The complex can then be treated to render it specific for the particular cells which it is desired to attack by bonding it covalently to a selected monoclonal antibody which displays specific immunobinding capacity for the particular cells. The covalent bonding of the complex to the monoclonal antibody can be carried out either before or after the complex is severed or cleaved from the support, using any of the conventional procedures or crosslinking agents, including those described by Thorpe et. al. loc. cit.

In one preferred embodiment, as illustrated in Example 1, the complex is linked through the ligand to the support via a disulfide group cleavable by reduction. The resulting residual thiol group left on the ligand is then used as a functional group to form a covalent bond with the monoclonal antibody. For this purpose any suitable bifunctional cross-linking agent capable of reacting with the thiol group and also with an amino or other group of the monoclonal antibody may be employed. Preferably a functional group reactive with the thiol group to form the covalent bond is introduced in the monoclonal antibody to enable it to react directly with the functional group of the complex. For example, when the functional group on the complex is a thiol, a monoclonal antibody may be reacted with a reagent such as succinimidyl 4-(N-maleimidomethyl)cyclohexane-1-carboxylate (SMCC) to provide a maleimido group on the antibody.

In another preferred embodiment, as illustrated in Example 2, the ligand (as well as the resultant lectin-ligand complex) is linked to the support through a photocleavable group, an ortho-nitrobenzylcarbamate cleavable by radiation, and there was employed a difunctional cross-linking reagent to form covalent bonds stable to radiation between the lectin and the ligand of the ligand-support probe.

In still another preferred embodiment, as illustrated in Example 3, the ligand is linked to the support via an azo group cleavable by preferential reduction with sodium dithionite, while the lectin-ligand covalent bond is formed by a difunctional cross-linking agent forming bonds inert to such reduction.

The following specific examples will serve to illustrate more fully the method of the present invention without acting as a limitation upon its scope.

### Example 1

### Preparation of Modified Support

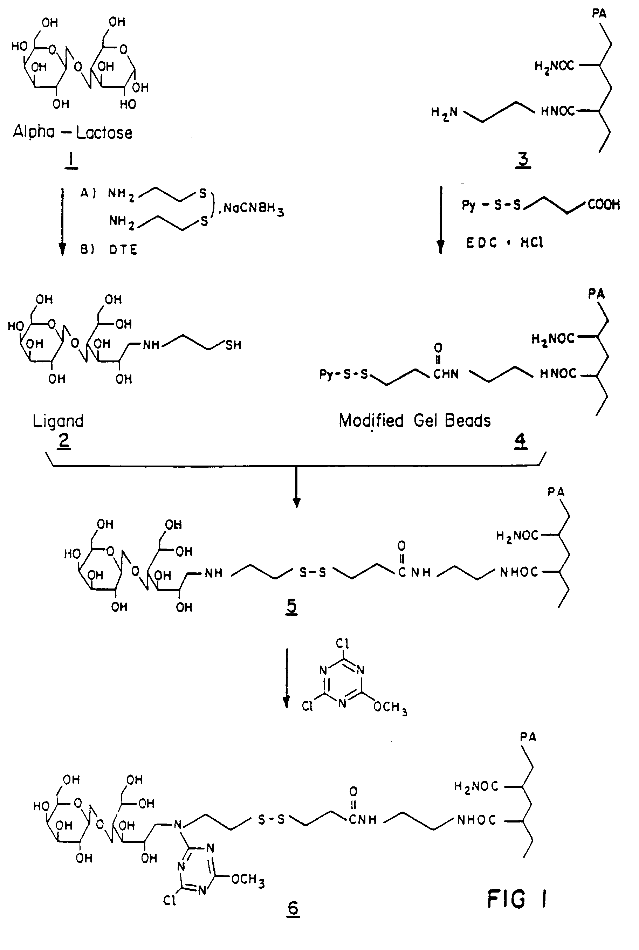

Aminoethylpolyacrylamide P-150 (Bio-Rad.) in the form of solid beads was treated to cap any residual carboxyl groups by reacting with a water-soluble carbodiimide in aqueous ammonium chloride as described by Inman, Methods Enzymology 34, 30-58 (1974). The beads were then functionalized with pyridyldithio groups which provide a point of attachment for the ligand via a disulfide linkage as shown by the reaction summary depicted at the upper right of Fig. 1. For this purpose, to a suspension of the carboxy-capped aminoethyl-polyacrylamide 3 (30 mL of packed beads) and 10 mL of a solution of 0.1 M sodium chloride was added .01 g (5 mmol) of 2-pyridyldithiopropionic acid (Py-S-S ∼ CO₂H) in 10 mL of dimethylformamide and 1 mL of water. Diluted hydrochloric acid was added to adjust the pH of the mixture to 4.7, and there was mixed with the suspension 5 mL of water containing 1.15 grams (6 mmol) of 1-ethyl-3-(3-dimethylaminopropyl) carbodiimide hydrochloride (EDC.HCl). After agitation for 7 hours at room temperature, a further 0.766 gram (4 mmol) of the carbodiimide was added and the pH of the mixture was readjusted with hydrochloric acid to 4.7. After shaking for 4 days at room temperature, the suspension was drained and the modified gel beads 4 were washed extensively with 0.2 M sodium chloride solution. To ensure complete removal of excess 2-pyridyldithiopropionic acid, the suspension was acidified with 0.1 M hydrochloric acid and extracted with 5 mL of ethyl acetate. The beads 4 were washed with 100 mM sodium phosphate buffer at pH 7.0. Washing was continued until a portion of the wash solution, when treated with excess dithioerythritol, showed a very low concentration of 2-pyridinethione, exhibiting an absorbance less than 0.1 a.u. at 343 nm.

The surface concentration of dithiopyridyl groups on the column of modified beads 4 was determined by reacting a sample of the beads with an excess of dithioerythritol (DTE) in a solution of 100 mM sodium bicarbonate at room temperature for 30 minutes. The beads were then washed repeatedly with a solution of 0.2 M sodium chloride and the amount of 2-pyridinethione in the washings, as measured by absorbance at 343 nm, (T. Stuchbury et al., Biochem. J., Vol. 151, p. 417-432 (1975)) was found to correspond to a concentration of 38 µ moles/ml of packed beads.

In order to cap any excess amino groups present on the 2-pyridyldithio-containing polymer beads 4, the beads (27 mL) were suspended in 50 mL of 100 mM sodium bicarbonate solution to which was added 4.8 g (5.2 mmol) methyl chloroformate at room temperature. After 5 minutes, excess chloroformate was removed by extraction with 20 mL ethyl acetate, and the modified gel beads were washed with 100 mM sodium phosphate buffer at pH 7.0.

### Preparation of Ligand

A ligand was prepared as depicted at the upper left of Fig. 1. There was dissolved in 36 mL of water 9.72 g (13.5 mmole) of alpha-lactose monohydrate 1. To the solution was added 3.68 g (13.5 mmole) of cystamine diacetic acid salt together with a solution in 72 mL methanol of 1.70 g (27.0 mmole) sodium cyanoborohydride. After adjusting the pH of the mixture to 6.2 with acetic acid, the solution was stirred at room temperature for 36 hours, at which time the methanol was removed by evaporation, the pH of the residual aqueous solution was adjusted to 5.0 with acetic acid, and the solution was applied to a 360 mL column of carboxymethylcellulose equilibrated in 2 mM pyridinium acetate buffer at pH 5.0. After washing the column with 2 volumes of the equilibrating buffer, the bound material was eluted with 0.1 M aqueous ammonia solution. The solutions containing eluted material were evaporated to dryness, and the solid redissolved in 200 mL of water to which was added 2.0 g (13.5 mmol) of dithioerythritol (DTE) at room temperature. After standing for one hour, the resulting solution containing a mixture of N-(2'-mercaptoethyl)lactamine 2 and cysteamine was applied to a 370 mL column of carboxymethylcellulose in 10 mM triethylammonium bicarbonate buffer at pH 7 containing 1 mM DTE. The column was washed with 500 mL of the same buffer, then developed with a linear gradient (1 L plus 1 L) of solution of 10-100 mM triethylammonium bicarbonate at pH 7 containing 1 mM DTE. Repeated evaporation from water and lyophilization produced 3.85 g (35% of the theoretical) of N-(2'-mercaptoethyl) lactamine ligand 2 as a white powder.

Immediately before use the ligand 2 prepared and purified as described above was treated to reduce any dimers formed between two molecules of lactamine, by dissolving 1.5 g (3.7 mmol) of the ligand in 10 mL of water at pH 8, adding 154 mg (1 mmol) of DTE and allowing to stand at room temperature for one hour. The pH was then lowered to 5.2 with acetic acid, the solution was applied to a 100 mL column of carboxymethylcellulose in 2 mM pyridinium acetate buffer, pH 5.2. The column was washed with 200 mL of the same buffer until all of the DTE had been removed. The pure N-(2'-mercaptoethyl) lactamine 2 was eluted in the free thiol form with 0.1 M hydrochloric acid. Upon assay with Ellman's reagent, the solution was found to contain 3.2 mmol of the thiol (85% of the theoretical).

### Preparation of Ligand-Support Probe

The ligand 2 was covalently linked to the previously prepared capped and activated polyacrylamide gel beads 4 by adding 50 mL (3.2 mmol) of the aqueous solution of the lactamine at pH 5 to 27 mL of the gel beads at room temperature, resulting in a suspension at pH 6.5 which was shaken at room temperature for 15 hours, then washed repeatedly with 0.2 M sodium chloride solution with successive centrifugation and decantation steps until the washings showed that substantially all of the 2-pyridinethione by-product had been removed, as indicated by measurements of the absorbance at 343 nm. The resulting beads, in the form of a probe 5 containing the ligand covalently linked to the polymeric beads had a surface lactose concentration of 38 mM.

In order to bind ricin covalently to the ligand-support probe 5 the probe was activated with the bifunctional cross-linking agent 2,4-dichloro-6-methoxytriazine by adding to a suspension containing 10 mL of the probe in 10 mL of a solution of 100 mM sodium bicarbonate, a solution containing 360 mg (2 mmol) of the triazine reagent in 10 mL dioxane. After vortexing the suspension for 1 minute, the excess triazine was removed by extraction with 4 mL of diethyl ether; the activated probe beads 6 were then equilibrated with 100 mM sodium phosphate buffer at pH 7.0 and packed into a column.

### Preparation of Lectin-Ligand-Support Complex

Ricin was applied to the activated probe column 6 in the form of 2.5 mL of a solution containing 2.1 mg/mL of ricin in 0.01 M sodium phosphate buffer, pH 7.0, containing 0.15 M NaCl. In order to form a covalent bond between the ricin and the second functional chlorine group of the triazine cross-linking agent, the column was then washed with three column volumes of 50 mM triethanolamine hydrochloride buffer at pH 8.6. After standing for 24 hours at room temperature at pH 8.6, the column was washed with 3 volumes of 0.5 M galactose solution at pH 7, in order to remove any ricin that had not formed a covalently bonded ricin-ligand complex.

### Severing of Lectin-Ligand Complex from Support

The covalently bonded ricin-ligand complex prepared as described above was severed from the support by cleavage of the disulfide group between the ligand and the polymer. It was found that this disulfide group was cleaved or severed more readily by reduction with 10 mM DTE at pH 7 than is the disulfide group joining the A-chain and B-chain of the ricin itself. Although a variety of concentrations of DTE at pH values from 7 to 8 were investigated, optimal results, with minimal cleavage of the ricin chains were obtained at a concentration of 10 mM DTE at pH 7.0 at room temperature for 45 minutes, producing a yield of 36% of the theoretical. The free complex thus obtained did not bind to asialofetuin, demonstrating that the oligosaccharide binding sites of the ricin were blocked.

The ricin-ligand complex prepared as described above was affinity purified on a column of concanavalin A-Sepharose in 100 mM sodium phosphate buffer, pH 6. The complex binds to concanavalin A via the carbohydrate chains of the ricin component, and can thus be freed of contaminants, in particular low molecular weight thiols that would interfere with the conjugation reaction. The ricin-ligand complex was eluted with 100 mM, sodium phosphate buffer, pH 6, containing 1 M methyl α-D-mannopyranoside, resulting in 12 mL of a solution in which there was 150-200 µg of ricin-ligand complex. With a specimen of the product, it was shown that the A-chain inhibited protein synthesis in reticulocyte lysates to the same extent as the A-chain derived from native ricin. That this material had been modified in the desired way was confirmed by its passage through a column of immobilized asialofetuin, which has been shown to have an association constant for ricin of approximately 0.1 µ M. By this criterion, the ricin binding site was considered to be blocked.

### Covalent Bonding of Lectin-Ligand Complex to Monoclonal Antibody

In order to provide the ricin-ligand complex with the desired specificity towards selected cells, it was linked to the murine monoclonal anti-CALLA antibody J5, which is commercially available from Coulter Inc., Hialeah, Florida. For the purpose of conjugation, J5 was purified and then modified with SMCC as described by Lambert et al., J. Biol. Chem., Vol. 260, 12035-12041 (1985).

The solution of ricin-ligand complex as described above was then mixed with the purified and modified J5 antibody in buffer at pH 7 and allowed to stand one hour at 4°C. After one hour, it was shown by SDS-PAGE that the reaction mixture contained a covalently linked conjugate between the monoclonal antibody and the ricin-ligand complex, as well as excess monoclonal antibody and some unreacted ricin-ligand complex. The reaction mixture was then subjected to an affinity purification on Protein A-Sepharose, in 100 mM sodium phosphate buffer, pH 7.0, which removed unreacted ricin-ligand complex. This step also removed the methyl α -D-mannopyranoside introduced earlier, thereby allowing the use of a column of concanavalin A-Sepharose as a further purification step. The material eluted from the Protein A-Sepharose column, which comprised conjugate and excess monoclonal antibody, was then applied to a column of concanavalin A-Sepharose, where, as previously, the conjugate was bound through the carbohydrate chains of the ricin-ligand complex. Excess monoclonal antibody was then removed by extensive washing, and the bound conjugate was eluted with 1 M methyl α-D-mannopyranoside in 100 mM sodium phosphate buffer, pH 7.0.

Both the ricin-ligand complex, and the complex bonded to the monoclonal antibody, were tested for cytotoxicity using Namalwa cells, a Burkitt's lymphoma line that expresses, on the average, 60,000 CALLA per cell, and more than 600,000 binding sites for ricin. The ricin-ligand complex was found to be about 10 times less toxic than native ricin. This reduction in nonspecific toxicity is due to the blocking of the binding sites on the B-chain, as it was shown that the A-chain, when liberated from the complex, was fully active (vide supra). When the ricin-ligand complex was linked to J5, its toxicity increased 3-fold for target cells expressing CALLA, and its toxicity decreased 5-fold for non-target cells that lack CALLA. Most significantly, the toxicity of the conjugate was reduced three-fold by addition of a saturating amount of J5, showing clearly that the antibody confers some specificity on the modified toxin.

### Example 2

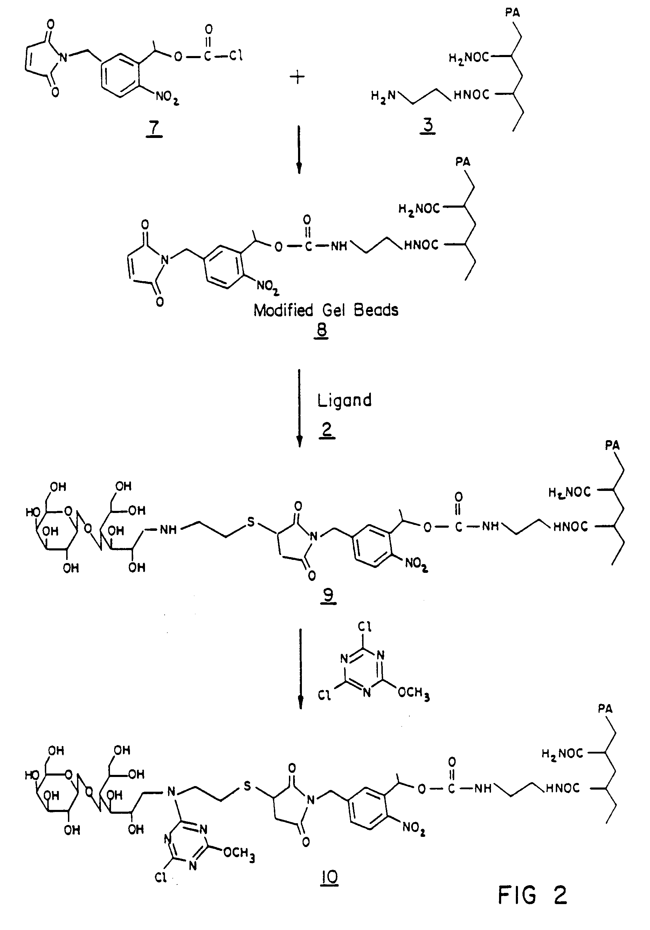

The reaction scheme for making the probe of this example is depicted in Fig. 2.

### Preparation of the Modified Support

To a suspension of carboxy-capped aminoethylpolyacrylamide P-150 3 prepared as described in Example 1 (26 mL of packed beads) in 0.1 M sodium bicarbonate (13 mL) was added 1-(5-maleimidomethyl-2-nitrophenyl)-ethyl chloroformate 7 (1.02 g, 3.48 mmol) in dioxane (6 mL). After 5 min of vigorous shaking, methyl chloroformate (5 mL) was added to cap any excess amino groups and shaking was continued for another 5 min. Modified polyacrylamide gel beads 8 were then recovered by filtration and washed successively with 0.1 M sodium phosphate buffer, pH 7.0, a mixture of 0.1 M sodium phosphate buffer, pH 7.0 and dimethylformamide (1:1, v/v), dimethylformamide and then with the same solutions in reverse order.

### Preparation of Ligand-Support Probe

The modified polyacrylamide beads 8 (24 mL of packed beads) were suspended in 0.1 M sodium phosphate buffer, pH 7.0 (10 mL) and treated with a solution of the ligand N-(2'-mercaptoethyl)lactamine 2 prepared as described in Example 1 (700 mg, 1.74 mmol) in water, pH 7.0. The mixture was shaken overnight and the probe 9 containing the ligand covalently linked to the polymeric beads was then recovered by filtration on a Büchner funnel and thoroughly washed with sodium phosphate buffer pH 7.0. A sample of the ligand-support probe 9 (0.3 mL) was used to prepare a small column and to measure the specific binding capacity of the probe beads for ricin. It was found that 1 mL of probe beads at pH 7.0 bound in excess of 1.0 mg of ricin in a specific fashion. All the specifically bound ricin could be eluted with buffer containing 0.2 M lactose.

A column of probe beads 9 was activated with the bifunctional cross-linking reagent 2,4-dichloro-6-methoxy-triazine in the following way. To a suspension of the polyacrylamide beads containing the lactose ligand (10 mL) in 0.1 M sodium bicarbonate (10 mL) was added a solution of 2,4-dichloro-6-methoxytriazine (0.24 g, 1.34 mmol) in dioxane (6.6 mL). The suspension was vigorously shaken for 1 min, and the excess triazine was then removed by extraction with diethyl ether (3 x 5 mL). The activated probe beads 10 were then equilibrated with 0.1 M sodium phosphate buffer, pH 6.5 and packed into a column.

### Preparation of Lectin-Ligand-Support Complex

A solution of ricin (10 mg in 5 mL of 0.01 M sodium phosphate buffer, pH 7.0 containing 0.15 M sodium chloride) was passed twice through the activated probe column, which was then washed with three column volumes of 0.05 M triethanolamine hydrochloride, pH 8.6 and left at ambient temperature for 24 hours. Ricin which was bound but not covalently crosslinked was removed from the beads by washing with 0.1 M sodium phosphate buffer, pH 7.0 containing 0.2 M lactose.

### Severing of Lectin-Ligand Complex from Support

Ricin retained by covalent linkage with the ligand was released from the support beads by photolysis in the following way. The beads were transferred from the column with 0.1 M sodium phosphate buffer (10 mL) to a glass petri dish where the suspension formed a layer of not more than 0.5 cm thickness. The suspension was then irradiated for 10 min at a distance of 15 cm from a Black Ray Longwave Ultraviolet Lamp (Model B-100 A, Ultraviolet Products, Inc., San Gabriel, CA, emission peak at 365 nm, intensity at 15 cm is approximately 1.1 mW/cm²) to cleave the linkage between the ligand and the support. The irradiated suspension was poured back into the column and the beads were thoroughly washed with 0.1 M sodium phosphate buffer, pH 7.0. The combined washings (50 mL), which contained the released ricin-ligand complex free from the support were passed through a column of asialofetuin-TSK (2 mL, binding capacity for ricin: 4 mg/mL) to remove traces of ricin contaminants that may have been present. The final solution was concentrated to 2 mL by ultrafiltration (YM-10 membrane, Amicon, Danvers, MA) and then passed through a small column of Biogel P-6 equilibrated in 0.05 M triethanolamine hydrochloride buffer, pH 8.0 containing sodium chloride (150 mM) and EDTA (1 mM), yielding 1.2 mg of pure ricin-ligand complex in 2.8 mL of buffer.

### Covalent Bonding of Lectin-Ligand Complex to Monoclonal Antibody

In order to link the complex covalently to the antibody J5, a sulfhydryl group was introduced into the complex with the heterobifunctional cross linking reagent 2-iminothiolane hydrochloride (Pierce Chemical Co., Rockford, IL) and a maleimido group was introduced into the monoclonal antibody with the heterobifunctional crosslinking reagent succinimidyl 4-(N-maleimidomethyl)cyclohexane-1-carboxylate (SMCC), (Pierce Chemical Co., Rockford, IL), as described in detail below.

The solution of ricin-ligand complex (2.8 mL) was cooled on ice and treated with a 0.5 M solution of 2-iminothiolane hydrochloride, pH 8.0 (0.044 mL), giving a final concentration of 2-iminothiolane of 8 mM. The reaction was stopped after 90 min on ice by gel filtration through a column of Biogel P-6 equilibrated with 5 mM Bistris-acetate buffer, pH 5.8, containing sodium chloride (50 mM) and EDTA (1 mM). In this way, an average of 0.8 thiol groups were introduced per molecule of ricin-ligand complex.

To a solution of J5 in 0.1 M sodium phosphate buffer, pH 7.0 (3 mg of JS in 1.5 mL) were added 12 equivalents of SMCC (0.075 mg) in dry dioxane (0.02 mL). After incubation at 30°C for 30 min., the reaction solution was passed through a column of Sephadex G-25 at 4°C, yielding modified J5 with an average of 1.5 maleimido groups per antibody molecule.

The solutions of modified ricin-ligand complex and modified J5 were mixed and the pH was adjusted to 7.0 by adding 0.5 M triethanolamine hydrochloride buffer, pH 8.0 (0.028 mL). After standing overnight at 4°C, iodoacetamide was added to a final concentration of 5 mM and the solution concentrated by ultrafiltration (YM-10 filter, Amicon) to about 2 mL. Purification was effected by gel filtration through a column (1.5 x 96 cm) of Biogel P-300 in phosphate buffered saline.

Both the ricin-ligand complex and the complex bonded to the monoclonal antibody displayed reduced non-specific toxicity characteristics similar to those of the products of Example 1.

### Example 3

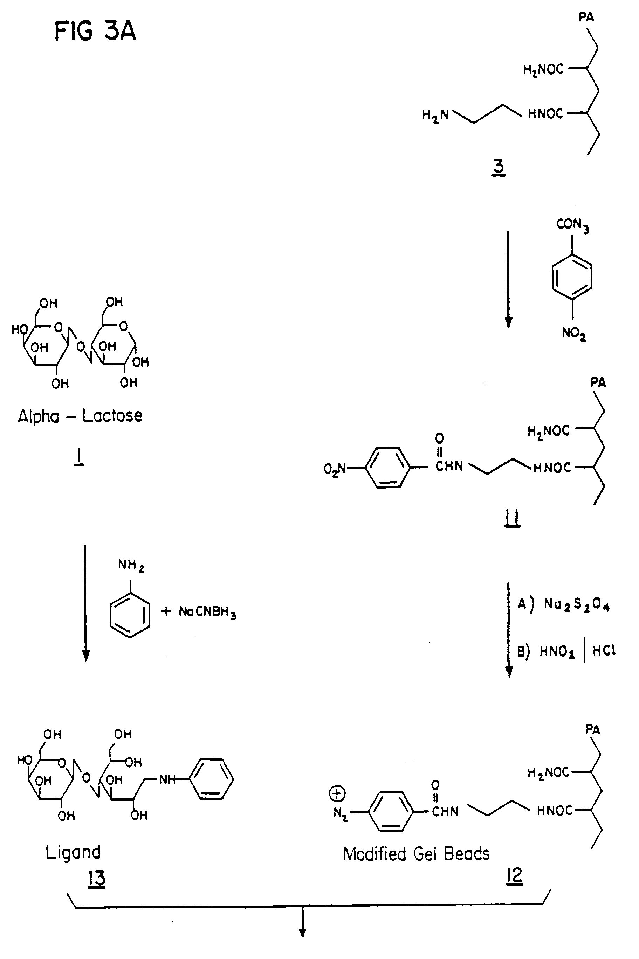

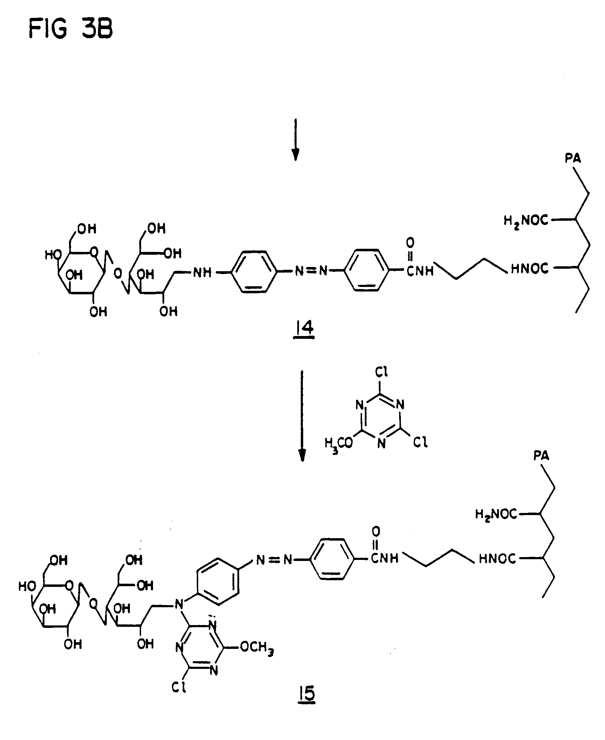

The ligand is linked via an azo functionality and can be released by treatment with 0.5 M sodium dithionite, pH 8.5 (see, for example, Cohen, Methods Enzymol., Vol. 34 (1974), 102-108). The reaction sequence for making the probe is summarized in Figs. 3A and 3B.

### Preparation of Ligand

To a stirred solution of -lactose (4.0 g, 11.1 mmol as the monohydrate) in water (40 mL) were added a solution of aniline (2.07 g, 22.2 mmol) in methanol (8 mL), and a solution of sodium cyanoborohydride (0.7 g, 11.1 mmol) in methanol (6 mL). The pH of the mixture was adjusted to 6.5 with acetic acid, and the solution was stirred at room temperature.

After 15 h, methanol was removed by evaporation, and acetone (12 mL) was added to destroy excess sodium cyano-borohydride, and the solution was stirred at room temperature for 1 hour. This solution was then evaporated to dryness, the residue redissolved in water (75 mL), the solution adjusted to pH 5.5 with acetic acid, and applied to a column (60 mL) of Amberlite IRA-118H ion-exchange resin, equilibrated in water. The column was washed with water (600 mL) and the sugar was eluted with a solution of 1 M ammonium bicarbonate, giving 5.4 g of a white solid which contained N-phenyllactamine ligand 13 contaminated with some salt.

### Preparation of Modified Support

To a suspension of carboxy-capped aminoethylpolyacrylamide P-150 3 (12 mL of packed beads) in a solution of 0.2 M NaCl (total volume 20 mL) was added p-nitrobenzoylazide (576 mg, 3 mmol) in THF (5 mL), and triethylamine (304 mg, 3 mmol). The gel was shaken gently for 20 minutes and a further 304 mg (3 mmol) of triethylamine was then added. After shaking the suspension for a further 25 minutes, the p-nitrobenzoyl-substituted support beads 11 were washed successively with a mixture of THF and 0.2 M NaCl solution (1:1, v/v), formamide, and 0.2 M NaCl solution.

The beads were then suspended in a solution or 100 mM NaHCO₃ (10 mL) and shaken vigorously with methyl chloroformate (2 mL) for 5 min. to cap any excess amino groups. The suspension was extracted with diethyl ether (2 x 15 mL) to remove excess chloroformate, and the beads were washed with 100 mM sodium phosphate buffer, pH 7.0. To a suspension of the gel in 100 mM sodium phosphate buffer, pH 7.0 (20 mL, final volume) was added sodium dithionite (2.61 g, 15 mmol). The suspension was then maintained at 55°C in a water bath, being removed and shaken periodically. After 45 min., the p-aminobenzoyl-substituted support gel beads were washed with a solution of 0.2 M NaCl. To a suspension of these modified polyacrylamide gel beads (5 mL of packed beads) in 1 M HCl (10 mL, final volume) at 0°C, was added a cold solution of sodium nitrite (69 mg, 1 mmol) in water (1 mL). The suspension was shaken gently at 4°C for 20 min. and the beads were then washed quickly with a cold solution of 0.2 M NaCl to provide p-diazobenzoyl-substituted support beads 12.

### Preparation of Ligand-Support Probe

A solution of the N-phenyllactamine ligand 13 (810 mg, 1.9 mmol) in a saturated solution of sodium borate (10 mL) was added to the cold diazo-modified support 12, which immediately became a deep red color. The suspension was shaken gently at 4°C overnight, then washed thoroughly with 0.2 M NaCl solution, leaving deep red colored ligand-support probe 14 in which the ligand was covalently bonded to the support through an azo group. A portion of the ligand-support probe 14 (0.50 mL) was used to prepare a small column with which the specific binding capacity was shown to be 0.5 mg of ricin per mL of packed beads. It was established that the ricin was bound specifically by showing that the bound ricin could be eluted in two ways. Either the ricin-ligand specific interaction could be disrupted by competition with a high concentration of galactose or the ligand-support bond could be cleaved by treatment with a solution of 0.2-0.5 M sodium dithionite pH 8. In both cases, ricin or ricin-ligand complex was eluted quantitatively from the column. As further proof that treatment with dithionite released ricin by cleavage of the ligand from the support, the gel immediately lost its deep red color, which is due to the chromophore of the azo group.

The ligand-support probe 14 was activated with the bifunctional cross-linking reagent 2,4-dichloro-6-methoxytriazine in the following way. To a suspension of the ligand-support probe beads 14 (5 mL volume packed beads in 100 mM sodium phosphate buffer, pH 7.0) in dry DMF (20 mL) was added a solution of 2,4-dichloro-6-methoxytriazine (1.8 g, 10.0 mmol) in dry DMF (10 mL). The suspension was agitated gently at room temperature for 22 hours. The activated probe beads 15 were then washed successively with DMF, DMF-100 mM sodium phosphate buffer, pH 7.0, and finally 100 mM sodium phosphate buffer, pH 7.0 and packed into a column.

### Preparation of Lectin-Ligand-Support Complex

A solution of ricin (5.7 mg in 3 mL 0.01 M sodium phosphate buffer, pH 7.0, containing 0.15 M sodium chloride) was applied to the activated probe column 15 and the flow-through reapplied twice. The column was washed with three column volumes of 0.05 M triethanolamine hydrochloride buffer, pH 8.6, and left at ambient temperature for 24 hours. Ricin which was bound but not covalently cross-linked was removed by washing the column with 0.1 M sodium phosphate buffer, pH 7.0, containing 0.5 M galactose. The ricin-ligand complex was then released from the support by severing the covalent bond in the following way. The column was washed with 0.05 M triethanolamine hydrochloride buffer, pH 8.6 to remove residual galactose, and then with a solution of 0.2 M sodium dithionite containing 0.3 M sodium chloride, pH 8.0. The beads were then transferred to a separate container and stirred with the solution of 0.2 M sodium dithionite, 0.3 M sodium chloride, pH 8.0 for 20 min. The suspension was filtered and the beads were treated twice more with dithionite in the same way. The dithionite solutions were pooled, giving a solution of 12 mL containing approximately 0.8 mg of ricin-ligand complex free from the support.

The complex can be bonded covalently to a monoclonal antibody such as J5 using conventional bifunctional crosslinking agents.

Other lectins can be substituted for ricin with similar results. The lectin-ligand-monoclonal antibody complexes can be used to kill selected cells in vitro as well as in vivo.

## Claims

1. A cytotoxic lectin complex having diminished non-specific toxicity compared to the free lectin comprising a cytotoxic lectin covalently bonded to a specific ligand comprising a disaccharide or higher polysaccharide derivative capable of binding specifically to an oligosaccharide-binding site on the lectin and a monoclonal antibody covalently bonded to the cytotoxic lectin complex.

2. A cytotoxic lectin complex according to claim 1 wherein the specific ligand comprises a galactose moiety.

3. A cytotoxic lectin complex according to claim 1 or claim 2 wherein the cytotoxic lectin is ricin or abrin.

4. A cytotoxic lectin complex according to any of claims 1 to 3 wherein the specific ligand is N-(2'-mercaptoethyl)-lactamine or N-phenyl-lactamine.

5. A specific ligand which binds specifically to and forms a covalent bond with an oligosaccharide binding site on a cytotoxic lectin, provided that the covalent bond to the oligosaccharide-binding site is not formed via a photoactivatable polysaccharide group.

6. A specific ligand according to claim 5 which is capable of forming a covalent bond to a monoclonal antibody.

7. A specific ligand according to claims 5 or 6 which comprises a polysaccharide derivative, preferably containing a galactose moiety, which is specific for the cytotoxic lectin.

8. A specific ligand according to any of claims 5 to 7 wherein the cytotoxic lectin is ricin or abrin.

9. A specific ligand according to any of claims 5 to 8 wherein the specific ligand is N-(2'-mercaptoethyl)-lactamine or N-phenyl-lactamine.
